(19)

Europäisches
Patentamt
European
Patent Office
Office européen
des brevets

(11)  **EP 4 388 877 A1**

(12)  **EUROPEAN PATENT APPLICATION**

(43) Date of publication:
**26.06.2024  Bulletin 2024/26**

(21) Application number: **22216428.7**

(22) Date of filing: **23.12.2022**

(51) International Patent Classification (IPC):
*A01N 63/20* (2020.01)    *A01N 63/22* (2020.01)
*A01P 1/00* (2006.01)    *C11D 3/38* (2006.01)

(52) Cooperative Patent Classification (CPC):
**A01N 63/20; A01N 63/22; A01P 1/00; C11D 3/381;**
**C12N 1/20; C12N 9/2417; C12N 9/54;**
C12R 2001/07

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **BRAIN Biotech AG**
**64673 Zwingenberg (DE)**

(72) Inventors:
• **Borgmeier,  Claudia**
**64673 Zwingenberg (DE)**

• **von Grote-Pastré, Julia**
**64625 Bensheim (DE)**
• **Bedue, Olivier**
**69469 Weinheim (DE)**

(74) Representative: **Fuchs Patentanwälte**
**Partnerschaft mbB**
**Tower 185**
**Friedrich-Ebert-Anlage 35-37**
**60327 Frankfurt am Main (DE)**

Remarks:
The complete document including Reference
Table(s) and the Sequence Listing(s) can be
downloaded from the EPO website

(54)  **MICROORGANISM CAPABLE OF FORMING A METABOLIC BIOFILM AND METHODS FOR PRODUCING THE SAME AND OF USING IT**

(57)    A microorganism, or spore thereof, capable of forming a metabolic biofilm is disclosed. Also, compositions, methods and uses of the microorganism, or spore thereof, are disclosed. (Figure 1)

Figure 1

EP 4 388 877 A1

**Description**

Background

Microorganisms, such as bacteria, fungi and algae can develop biofilms on surfaces. The development of biofilms initially begins completely harmlessly: individual cells of microorganisms attach themselves to surfaces and multiply in the moist environment. Some bacteria are able to build up a layer of mucus, which on the one hand serves as protection against environmental influences and on the other hand enables the organisms to accumulate nutrients even from a very nutrient-poor environment.

However, during their life cycle the microorganisms produce waste products that are released from the cell to the outside. Depending on the environment, these waste products can cause discomfort in the environment (such as for example malodour) or even cause health problems in animals and/or humans.

Conventional countermeasures against such harmful biofilms mostly focus on the eradication of the microorganisms including their biofilm, which, however, results in a tedious cleaning process due to the resistance of some microorganisms to chemicals, heat and other eradication methods. The cleaning process has to be repeated on a regular basis and, due to the invisibility of the microorganisms and the biofilm, the successful cleaning cannot be controlled easily. Furthermore, surfaces which are not easily accessible, form retreats for the microorganisms, so that accelerated regrowth from those niches occurs and a full eradication of the biofilm becomes impossible. One attempted solution is to resort to ever more aggressive cleaning methods, which of course have negative effects on the environment and may cause harm to animals or humans.

Therefore, the need existed to provide means and/or methods for preventing the negative effects of microorganisms, such as for example malodour or health risks, on surfaces, which are less damaging to the environment and harmless to animals and humans. These means and/or methods are provided by the present disclosure.

Summary of disclosure

In a first aspect, the disclosure pertains to a microorganism, or spore thereof, capable of forming a metabolic biofilm.

Surprisingly, it has been found that certain microorganisms, or spores thereof, are capable of forming metabolic biofilms, i.e., a biofilm which can quickly degrade microbial waste products within the biofilm, which otherwise would cause discomfort (e.g., malodour) or even health problems. As such, the disclosed microorganisms, or spores thereof, provide a novel approach to controlling the microbial waste products within the biofilm without significantly changing the microbiotic diversity. The advantage of this approach is that the biofilm stays on the surface for a long time, once the microorganisms, or spores thereof, are applied; and disinfectants, detergents or other potentially harmful substances (for animals or humans) are not needed. Furthermore, since in a an embodiment, the microorganism, or spore thereof, shares the abilities to withstand heat or chemical treatments as well as multiple washing steps with the other microorganisms in the biofilm, the treatment has a long-lasting, self-renewable property, which cannot be achieved by conventional methods (i.e., conventional cleaning agents and the like).In a second aspect, the disclosure pertains to a method of producing a microorganism, or spore thereof, capable of forming a metabolic biofilm, comprising the steps of:

    a. Selecting a biofilm forming microorganism, or spore thereof,

    b. Producing an expression vector comprising at least one gene of which the gene-product has a metabolic activity selected from amylase and/or protease activity,

    c. Transforming the microorganism, or spore thereof, with the expression vector either stably and/or transiently,

    d. Isolating the microorganism, or spore thereof, thereby producing a microorganism, or spore thereof, capable of forming a metabolic biofilm.

In a third aspect, the disclosure relates to the use of a microorganism, or spore thereof, (i) for inhibiting or reducing health risks and/or discomfort in the environment by producing a metabolic biofilm in households, during food processes, during fermentation processes, during pharmaceutical processes, in public health environments and/or (ii) for inhibiting and/or reducing malodour, in particular by producing a metabolic biofilm.

Human-made articles and surfaces in households (e.g., in the kitchen, in the toilet), food processes (e.g., food production), fermentation processes (e.g., protein production in fermenters, cheese making, beer brewing, etc.), pharmaceutical processes (e.g., such as processes in the pharmaceutical industry) and/or public health environments (e.g., such as in hospitals, schools, kinder gardens, nursing homes, etc.) as well as the inhibition of malodour (e.g. in kitchen or bath utensils) are especially sensitive areas when it comes to conventional treatments with disinfectants, detergents or other, for human and animals potentially harmful substances. The disclosed use avoids the use of disinfectants, detergents or other, potentially harmful substances (for human and animals) and therefore provide an improvement over the prior art. Furthermore, treatment in said areas is a cumbersome task, since with conventional methods all areas comprising a biofilm need to be treated, whereas within the disclosed microorganism, or spore thereof, the application to one part of the area is sufficient, since the microorganism, or spore thereof, may expand its biofilm even to areas which were not originally treated.

In a fourth aspect the present disclosure relates to uses of the microorganism, or spore thereof, for inhibiting or reducing malodour on a surface as compared to a surface not comprising the metabolic biofilm, optionally corresponding to an odour difference of at least 0.3 grades, of at least 0.5 grades, or at least 1.0 grade in the VDA odour test.

In a fifth aspect, this disclosure relates an article comprising the microorganism, or spore thereof and/or comprising a metabolic biofilm.

In a sixth aspect, this disclosure relates to a composition comprising the microorganism, or spore thereof, and at least one further excipient, such as for example a buffer, a salt, a preservative, a deposit aid, a pharmaceutical excipient and/or a carrier.

Detailed description

The microorganism, or spore thereof, may be selected from a group consisting of at least one member of the *Bacillus subtilis group,* wherein the member of the *Bacillus subtilis group* is selected from the group consisting of *Bacillus amyloliquefaciens, Bacillus tequilensis, Bacillus subtilis, Bacillus atrophaeus, Bacillus vallismortis, Bacillus licheniformis, Bacillus sonorensis, and Bacillus mojavensis;* and/or a member of the *Priestia* (former *Bacillus)* megaterium, and/or a member of *Bacillus velezensis,* and/or a member of *Bacillus pumilus,* and/or a combination thereof.

The term *"Bacillus subtilis group"* as used herein means a sub-genus of the family *Bacillaceae,* genus *Bacillus,* comprising the species *Bacillus amyloliquefaciens, Bacillus tequilensis, Bacillus licheniformis, Bacillus subtilis, Bacillus atrophaeus, Bacillus vallismortis, Bacillus licheniformis, Bacillus sonorensis* and *Bacillus mojavensis subgroup.*

One of the microbial organisms of the *Bacillus subtilis group* is *Bacillus subtilis,* known also as the hay bacillus or grass bacillus, a Gram-positive, catalase-positive bacterium, found in soil and the gastrointestinal tract of ruminants and humans. As member of the genus *Bacillus, B. subtilis* is rod-shaped, and can form a tough, protective endospore, allowing it to tolerate extreme environmental conditions. *B. subtilis* has historically been classified as an obligate aerobe organism, though evidence exists that it is a facultative anaerobe. *B. subtilis* is considered the best studied Gram-positive bacterium and a model organism to study bacterial chromosome replication and cell differentiation.

The microbial organism of the present disclosure may be used in a vegetative form and/or as spores. Spores are resistant structures used by microorganisms for survival under unfavorable conditions. The ability to prepare spores and vegetative cells is considered routine in the art (Probiotics Antimicrob. Proteins, 2019 Sep; 11(3):731-747). The term "spore" or "spore thereof" is used hereinafter for bacterial spores which are resistant structures used for survival under unfavourable conditions by the bacteria. In some publications the synonym "endospore" may be used.

The microorganism, or spore thereof, may be isolated. In an embodiment, the microorganism or spore thereof is a probiotic microorganism or spore thereof.

Optionally, the microorganism, or spore thereof, is included in the list of QPS status recommended biological agents for safety risk assessments by the European food safety authority (ESA) as QPS-EFSA List 10.2903/j.efsa.2022.7408. This has the advantage that the microorganism itself does not represent any health risk and may be even used on surfaces of food-contact materials or in other health-sensitive areas. Alternatively, the microorganism, or spore thereof may fulfil the requirements necessary to be included in the QPS list.

In a further embodiment, the microorganism, or spore thereof, is heat-resistant, resistant to washing-drying cycles, hot washing, heat and/or to chemical stresses. Optionally, it passes at least one, at least two, at least three, or all four tests selected from:

a. a hot washing test with a ratio of less than 100,

b. a heat test with a ratio of less than 100,

c. a chemical stress test with a ratio of less than 100,

d. an amylase long-term polymer matrix test with a ratio of at least 1.5, and/or

e. a protease long-term polymer matrix test with a ratio of at least 1.5.

In an embodiment, the microorganism, or spore thereof passes the hot washing test to the extent that the ratio is less than 100, less than 50, or less than 20.

In an embodiment, the microorganism, or spore thereof passes the heat test to the extent that the ratio is less than 100, less than 50, or less than 20.

In an embodiment, the microorganism, or spore thereof passes the chemical stress test to the extent that the ratio is less than 100, less than 50, or less than 20.

In an embodiment, the microorganism, or spore thereof passes the amylase long-term polymer matrix test to the extent that the ratio is at least 1.5, at least 4, or at least 8.

In an embodiment, the microorganism, or spore thereof passes the protease long-term polymer matrix test to the extent that the ratio is at least 1.5, at least 4, or at least 8.

Definitions

These and other standardized tests are further defined hereinafter:

**Capability of forming a metabolic biofilm**

A biofilm with metabolic activity is determined by the increased activity to convert resazurin to fluorescent resorufin as compared to a standard biofilm-forming microorganism *Bacillus subtilis* (Ehrenberg 1835) Cohn 1872; DSM-No. 402. Optionally, the metabolic activity in the biofilm is increased by at least 2 times, at least 3 times, at least 4 times, 5 times, at least 10 times, as compared to the standard microorganism *Bacillus subtilis* (Ehrenberg 1835) Cohn 1872; DSM-No. 402 ("DSM402" hereinafter).

The microorganism, or spore thereof as disclosed herein is defined to be capable of forming a metabolic biofilm, if the microorganism or spore scores a fluorescence which is at least twice as high as a fluorescence scored by DSM402 under the same experimental conditions.

The experimental conditions include

- sterilizing polyurethane (PU) foam cubes (1x1x1 cm, 25 mg) in an autoclave at 121°C for 20 minutes;

- putting the cubes into a deep well plate (DWP), one cube per well;

- filling 2 ml of sterilized medium into each of the wells that contain a cube, wherein the medium is composed of skim milk powder 0.8 g/L, soluble starch 4 g/L and olive oil 0.6 g/L;

- after 5 minutes, taking out cubes from DWP and transferring them to empty well of new DWP;

- inoculating each cube with 10 $\mu$l of inoculation solution, i.e. containing microorganisms or spores in PBS buffer (e.g. $10^5$ CFU of microorganism or spore under examination and the same amount of DSM402 *[Bacillus subtilis* (Ehrenberg 1835) Cohn 1872; DSM-No. 402]; at least 4 cubes each); additionally, leaving four cubes without inoculation as control;

- incubating the inoculated cubes for 7 days at 20°C;

- after 48 hours and 96 hours within those 7 days, applying additional 2 ml of medium to each well and transferring each cube to new well;

- after the 7 days, transferring cubes to a new DWP and washing by application of 4 ml saline (0.9%) to each well; transferring cubes into new DWP; repeat washing and transferring step an additional two times (three times in total);

- after washing, adding 2 ml of a resazurin-containing solution (e.g. PrestoBlue by Thermo Fisher Scientific, 10 % in medium) dye solution to the cubes in each well and incubating for 6 hours at 37°C;

- transferring 150 $\mu$l of each well into a microtitre plate (e.g., Costar 3904, Corning) and detecting fluorescence (e.g. using Fluostar, BMG) with the following parameters: excitation wavelength 440-412 nm, emission wavelength 590 nm, gain 15, prior mixing (5 sec, 1 mm orbital).

The mean values (MV) of fluorescence (arbitrary fluorescence units) are determined for each microorganism or spore and for control; MV of non-inoculated control is subtracted. Microorganisms and spores under investigation scoring at least twice the fluorescence value of DSM402 are capable of forming a metabolic biofilm.

## Determination of amylase activity

Amylase activity is based on absorbance detection of a product of the underlying enzymatic reaction. Absolute and relative amylase activity can be determined.

The absolute amylase activity score is determined by an absorbance at 620 nm (cf. table 1 below). The test (Phadebas AB, Sweden) is based on a water-insoluble, cross-linked starch polymer substrate which is hydrolyzed by $\alpha$-amylase in the biofilm to form water-soluble blue fragments. The absorbance of the blue solution is a function of the $\alpha$-amylase activity in the sample.

The amylase activity test may be performed as follows:

- sterilizing polyurethane (PU) foam cubes (1x1x1 cm, 25 mg) in an autoclave at 121°C for 20 minutes;

- putting the cubes into a deep well plate (DWP), one cube per well;

- filling 2 ml of sterilized medium into each of the wells that contain a cube, wherein the medium is composed of pancreatic casein digest 10 g/L (e.g. Bacto Trypton, Thermo Fisher Scientific), NaCl 5 g/L, yeast extract 5 g/L (e.g. Bacto Yeast Extract, Thermo Fisher Scientific); glycerol 1%, MnSO$_4$ 0.1 mM (filter sterilized and supplemented after autoclaving);

- after 5 minutes, taking out cubes from DWP and transferring them to empty well of new DWP;

- inoculating each cube with 10 $\mu$l of inoculation solution, i.e. containing microorganisms or spores in PBS buffer (e.g. $10^5$ CFU of microorganism or spore under examination and the same amount of DSM402; at least 4 cubes each); additionally, leaving four cubes without inoculation as control;

- incubating the inoculated cubes for 20°C;

- after the 72 hours, transferring cubes to a new DWP and washing by application of 4 ml saline (0.9%) to each well; transferring cubes into new DWP; repeat washing and transferring step an additional two times (three times in total);

- after washing, adding 2.5 ml of an amylase-test-solution (e.g. Phadebas test solution; e.g. 9 Phadebas tablets dissolved in 65 ml of 2.4 g/L starch solution, sterilized by autoclaving) to the cubes in each well and incubating in a shaker for 2 hours at 37°C (e.g. Titramax, e.g. at shaking speed 7);

- transferring 200 $\mu$l of each well into 1.5 ml reaction tubes and centrifuging (5 min, 1500xg);

- transferring supernatant samples (100 $\mu$l) into 96 well microtitre plate (MTP) and measuring absorbance at 620 nm against deionized water; subtracting values measured for non-inoculated samples.

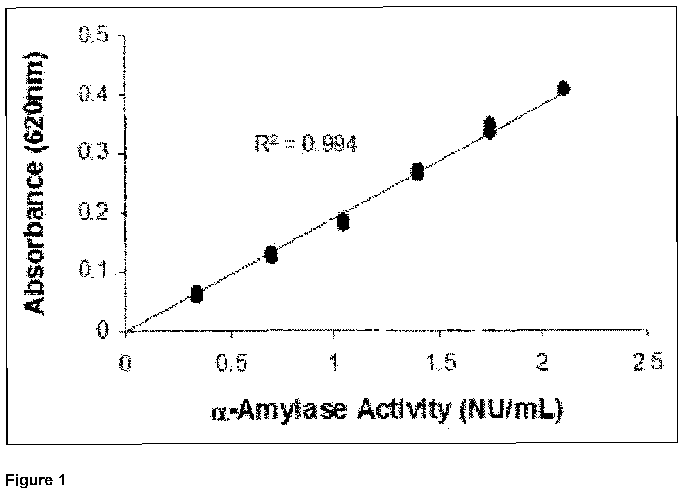

Figure 1 shows that there is a linear relationship between absorbance and enzymatic activity.

The absolute amylase activity score is determined in accordance with Table 1.

Table 1 - Comparison of absorbance and amylase activity score

| Absorbance | amylase activity score |
|---|---|
| 0 - 0.25 | 0 |
| 0.25 - 0.349 | 1 |
| 0.35 - 0.449 | 2 |
| 0.45 - 0.549 | 3 |
| 0.55 - 0.649 | 4 |
| 0.65 - 1 | 5 |

The relative amylase activity is determined by calculating the ratio of the absorbance-result of the microorganism or spore under investigation and that of DSM402 For example, an absorbance-result of 0.25 of the sample and an absorbance-result of 0.05 of DSM402 will result in 0.25/0.05, i.e., a relative amylase activity of 5:1.

The PU foam cubes used in this and the other tests discussed herein, may be selected from standard household sponges, e.g. made of soft polyurethane having a spatial weight 20 kg/m$^3$ and having about 20 pores cm$^{-1}$.

**Determination of protease activity**

Protease activity is based on absorbance detection of a product of the underlying enzymatic reaction. Absolute and relative protease activity can be determined.

The absolute protease activity score is determined by an absorbance at 440 nm of (cf. table 3 below). The test (Azocasein-test; Megazyme, Ireland) is based on an azocasein (azo-casein) substrate solution. The substrate is hydrolyzed by all endo-proteases which are active on casein to form a blue dye. The absorbance of the blue solution is a function of the protease activity in the sample.

The protease activity test may be performed as follows:

- sterilizing polyurethane (PU) foam cubes (1x1x1 cm, 25 mg) in an autoclave at 121°C for 20 minutes;

- putting the cubes into a deep well plate (DWP), one cube per well;

- filling 2 ml of sterilized medium into each of the wells that contain a cube, wherein the medium is composed of skim milk powder 0.8 g/L, soluble starch 4 g/L and olive oil 0.6 g/L;

- after 5 minutes, taking out cubes from DWP and transferring them to empty well of new DWP;

- inoculating each cube with 10 μl of inoculation solution, i.e. containing microorganisms or spores in PBS buffer (e.g. 10$^5$ CFU of microorganism or spore under examination and the same amount of DSM402; at least 4 cubes each); additionally, leaving four cubes without inoculation as control;

- incubating the inoculated cubes for 7 days at 20°C;

- after 48 hours and 96 hours within those 7 days, applying additional 2 ml of medium to each well and transferring each cube to new well;

- after the 7 days, transferring cubes to a new DWP and washing by application of 4 ml saline (0.9%) to each well; transferring cubes into new DWP; repeat washing and transferring step an additional two times (three times in total);

- after washing, adding 2.5 ml of Azo-Casein (Megazyme, Ireland) solution to the cubes in each well, e.g. azo-casein solution may be prepared by mixing 26 ml medium (as above) and 39 ml azo-casein (0.8 g Azo-Casein powder reconstituted according to Manufacturer's instructions);

- incubating in a shaker for 6 hours at 37°C (e.g. Titramax, e.g. at shaking speed 7);

- transferring 150 μl of each well into 1.5 ml reaction tubes, adding 450 μl of TCA (5%) and centrifuging (5 min, 1500xg);

- transferring supernatant samples (150 μl) into 96 well microtitre plate (MTP) and measuring absorbance at 440 nm against deionized water; subtracting values measured for non-inoculated samples.

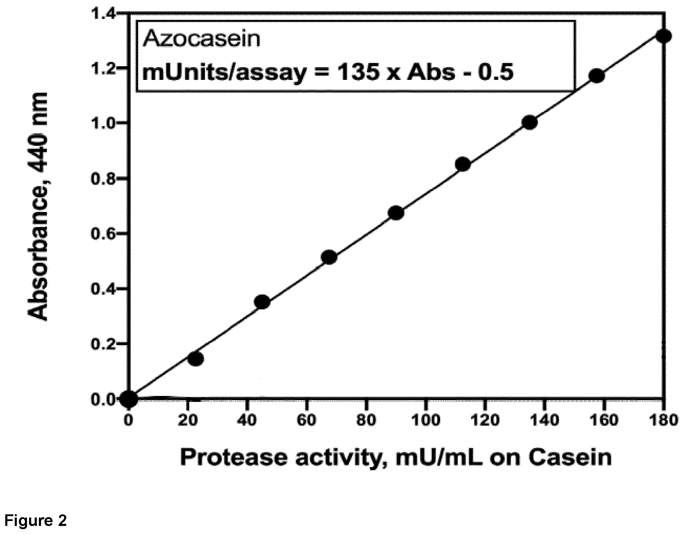

Figure 2 shows that there is a linear relationship between absorbance and enzymatic activity.

The absolute protease activity score is determined in accordance with Table 2.

The protease activity score then can be deduced from table 2.

Table 2. Comparison of absorbance and protease activity score

| absorbance | protease activity score |
|---|---|
| 0 - 0.25 | 0 |

(continued)

| absorbance | protease activity score |
|---|---|
| 0.25 - 0.349 | 1 |
| 0.35 - 0.449 | 2 |
| 0.45 - 0.549 | 3 |
| 0.55 - 0.649 | 4 |
| 0.65 - 1 | 5 |

The relative protease activity is determined by calculating the ratio of the absorbance-result of the microorganism or spore under investigation and that of DSM402 For example, an absorbance-result of 0.25 of the sample and an absorbance-result of 0.05 of DSM402 will result in 0.25/0.05, i.e., a relative protease activity of 5:1.

**CFU determination from surfaces and volumes**

To measure the concentration of colony forming units (CFU) on **surfaces** of an article, the microorganisms, or spores thereof, are sampled from the surface of the article by washing sections the surface of the article in 10 ml PBS-buffer per 1 $cm^2$ surface area. The collected buffer solution is heat treated in a water bath at 80°C for 5 minutes.

The $CFU/cm^2$ can be determined by plating the 10 ml PBS-buffer on sterile tryptic soy agar plates (e.g. TSA-standard-plates) and incubating at 37°C for 24h. Colonies can then be counted and the $CFU/cm^2$ can be determined for each of the sections on that surface.

To measure the CFU in **volumes** of an article, microbial organisms, or spores thereof, are sampled from the article by immersing a section of 1 $cm^3$ of the article in 10 ml PBS-buffer. The collected buffer solution is heat treated in a water bath at 80°C for 5 minutes.

The 10 ml PBS-buffer solution is then plated on ready-to-use tryptic soy agar monitoring contact plates, e.g. TSA with LTH-Thio cont.-ICR+ (Merck-Millipore, USA) at 37°C for 24h. Colonies can then be counted and the $CFU/cm^3$ can be determined for each of the sections of that article.

The specific strain of the microbial organisms, or spores thereof, can be determined by the gDNA determination described below.

**gDNA determination**

After isolation and incubation of the contact plates for 24 h at 37°C as described before, colonies are harvested for identification with sterile 10 $\mu$l inoculating loops (Neolab Migge GmbH, Germany) and resuspended in 20 $\mu$l bacterial protein extraction reagent, e.g. aqueous nonionic detergent solution comprising 20 mM Tris-HCl at pH 7.5 including lysozyme and DNase (e.g. B-Per reagent, FisherScientific, USA).

After incubation for 15 min at 37°C, 30 $\mu$l MilliQ water are added and 1 $\mu$l of the mixture applied in a colony PCR targeting the *groEL* locus (Biosci Biotechnol Biochem.; 1992 Dec;56(12):1995-2002; doi: 10.1271/bbb.56.1995) as follows:

- 7.5 $\mu$L PCR Master Mix, e.g. Phusion Flash Mastermix (FisherScientific, Germany)

- 1 $\mu$L Primer groEL 60-A, 10 pmol/$\mu$l (5'-ACCCTGGGCCCGAGGGCCGCAACGTCGT) (Seq ID. No. 1)

- 1 $\mu$L Primer groEL 160-B, 10 pmol/$\mu$l (5'-TGACCGCGACGCCGCCCGCCAGCTTCGC) (Seq ID. No. 2)

- 4.5 $\mu$L MilliQ-Wasser

- 1 $\mu$L bacterial protein extraction reagent, e.g. aqueous nonionic detergent solution comprising 20 mM Tris-HCl at pH 7.5 including lysozyme and DNase (e.g. B-Per reagent, FisherScientific, USA)

The following PCR-protocol is used:

| PCR-Cycles | |
|---|---|
| Temperature | Minutes |
| 94°C | 01:00 |
| -----30 cycles of------ | |
| 94°C | 00:10 |
| 55°C | 00:20 |
| 72°C | 00:20 |
| --------------------- | |
| 72°C | 01:00 |
| 10°C | hold |

The 1.1 kb PCR product is subsequently sequenced for strain identification. By doing so, gDNA per $cm^3$ or per $cm^2$ as well as strain diversity can be determined.

**Hot washing test**

The number of CFUs/$cm^2$ of the microbial organism, or spores thereof, on a surface of 1 $cm^2$; or CFUs/$cm^3$ of the microbial organism, or spores thereof, in volume of 1 $cm^3$ is determined according to the CFU-determination explained above. The surface or volume comprising the microbial organism, or spores thereof, is then washed in 10 ml PBS-buffer with a temperature of 80°C for 5 minutes.

Then the number of CFUs/$cm^2$ (or CFUs/$cm^3$) of the microbial organism, or spores thereof, is determined as shown above. The ratio between CFUs before and after the washing step is calculated.

Ratio = CFUs/$cm^2$ (or CFUs/$cm^3$) before washing step / CFUs/$cm^2$ (or CFUs/$cm^3$) after washing step.

If the ratio is less than 100, less than 50, or less than 20 the microbial organism, or spores thereof, has passed the hot washing test.

**Heat stress test**

The number of CFUs/$cm^2$ of the microbial organism, or spores thereof, on a surface of 1 $cm^2$; or CFUs/$cm^3$ of the microbial organism, or spores thereof, in volume of 1 $cm^3$ is determined according to the CFU-determination explained above.

The surface or volume comprising the microbial organism, or spores thereof, is then heated in a microwave for 5 minutes at 1000 Watt.

Then the number of CFUs/$cm^2$ (or CFUs/$cm^3$) of the microbial organism, or spores thereof, is determined again according to the CFU-determination explained above. The ratio between CFUs before and after the heating step is calculated.

Ratio = CFUs/$cm^2$ (or CFUs/$cm^3$) before the heating step / CFUs/$cm^2$ (or CFUs/$cm^3$) after the heating step.

If the ratio is less than 100, less than 50, or less than 20 the microbial organism, or spores thereof, has passed the heat test.

**Chemical stress test**

The number of CFUs/$cm^2$ of the microbial organism, or spores thereof, on a surface of 1 $cm^2$ or CFUs/$cm^3$ of the microbial organism, or spores thereof, in volume of 1 $cm^3$ is determined according to the CFU-determination explained above. The surface or volume comprising the microbial organism, or spores thereof, is then washed in 10 ml of PBS with 1 wt.-% hydrogen peroxide for 5 minutes.

Then the number of CFUs/$cm^2$ (or CFUs/$cm^3$) of the microbial organism, or spores thereof, is determined again according to the CFU-determination explained above. The ratio between CFUs before and after the washing step is calculated.

Ratio = CFUs/$cm^2$ (or CFUs/$cm^3$) before washing step / CFUs/$cm^2$ (or CFUs/$cm^3$) after washing step.

If the ratio is less than 100, less than 50, or less than 20 the microbial organism, or spores thereof, has passed the chemical stress test.

**Long-term polymer-matrix test**

The "long-term polymer-matrix test" is performed to evaluate whether a microorganism or spore thereof is capable of maintaining amylase or protease activity even after a treatment that resembles actual use of an article, i.e. including multiple wet and dry cycles. The test is performed using cubes of 20 g of a sponge polymer-material made from soft polyurethane (PUR) foam material (spatial weight 20 kg/m$^3$, having 20 pores cm$^{-1}$). The test compares amylase or protease activity after the cycles with the activity of non-inoculated cubes.

For the long-term polymer-matrix test ratio of the microbial organism, or spores thereof, one half of the sponge polymer-matrix cubes is treated with 10 ml of a PBS-solution comprising about 1 * 10$^5$ CFU/cm$^3$ of the microbial organism, or spores thereof, *("sample"),* the other half of the sponge polymer-matrix cubes is not inoculated (*"control"*).

Then each of the sponge polymer-matrix cubes (sample as well as control) is subjected to 45 wash and dry cycles.

Each wash and dry cycle consists of the following consecutive steps:

a. submerging the sponge polymer-matrix cube for 2 minutes in washing liquid (composition below) at a temperature of 45°C;

b. squeezing the sponge polymer-matrix cube three times while submerged in the washing liquid; wherein the cube is squeezed so far that the volume is reduced during squeezing by at least 50% of the volume of the material in "relaxed" (i.e., "un-squeezed" condition);

c. squeezing the polymer-matrix cube one time outside the liquid; (squeezing outside the liquid is done until the cube has a weight that exceeds its dry weight before having been submerged in washing liquid by more than 100%, but less than 150%;

d. placing the sponge polymer-matrix cube on a draining rack at 20°C for 24 hours.

The washing liquid, as mentioned above, consists of 5 L water, 3 ml Triton® X 100 (4-(1,1,3,3-Tetramethylbutyl)-phenyl-polyethylenglykol), and 25 ml of a standard broth medium comprising:

- enzymatic digest of soybean 3.0 g/L (tryptic digest)
- sodium chloride 5.0 g/L
- dipotassium hydrogen phosphate 2.5 g/L
- glucose monohydrate 2.5 g/L
- wherein the standard broth medium is autoclaved before use at 121°C for 15 minutes;
- and is adjusted to a final pH: 7.3 ± 0.2 at 25°C.

At the end (after the last drying step) of the long-term polymer-matrix test, samples of the sponge polymer cubes are directly transferred into deep well plates and amylase and protease activity both of the "sample" as well as the "control" is determined as explained above.

The ratio of the long-term polymer-matrix test is then calculated from the resulting activity values as follows:

ratio of the amylase long-term polymer-matrix test = amylase activity ("sample") / amylase activity ("control")

ratio of the protease long-term polymer-matrix test = protease activity ("sample") / protease activity ("control").

A microbial organism, or spore thereof, is considered to pass the amylase long-term polymer-matrix test if the ratio is at least 1.5, at least 2, at least 3, at least 4, at least 5, at least 6, at least 7, at least 8, at least 9, at least 10.

A microbial organism, or spore thereof, is considered to pass the protease long-term polymer-matrix test if the ratio is at least 1.5, at least 2, at least 3, at least 4, at least 5, at least 6, at least 7, at least 8, at least 9, at least 10.

**VDA odour test**

Articles or surfaces, for example sponges, with or without the microbial organism, or spores thereof, were evaluated using an odour test which was performed essentially as described in the standard procedure VDA 270 (*"Bestimmung des Geruchsverhaltens von Werkstoffen der Kraftfahrceug-Innenausstattung"*).

Samples are prepared according to the procedure described above for the long-term polymer matrix test. This means that the sponges have undergone the wash and dry cycles as described above, including step d. After that, the odour test is performed.

The odour test is performed at temperatures of 23°C +- 2°C. At least 24 hours before the test the dried sponges are kept at the same temperature in closed glass jars.

The procedure may be summarized as follows:

- Articles or surfaces, for example sponges, are placed in 250 ml clean glass jars with odourless sealing and screw cap
- Odour test is performed by at least 3 subjects (non-smoker, use of perfume or scented hand lotion precluded on that day) within one week; the same samples are tested by all subjects
- at least 3 reference samples are included in the test to assure that smelling capability of subjects are not restricted and to enable comparison between different odour tests.

The at least 3 subjects then grade the samples with grades from 1 to 6 (1 being the best), wherein the following grading principle is applied:

- Grade 1: smell not recognizable
- Grade 2: recognizable smell, but non-disturbing
- Grade 3: clearly recognizable smell, but still non-disturbing
- Grade 4: disturbing smell
- Grade 5: strong disturbing smell
- Grade 6: unbearable smell

A sample passes the VDA-odour test when the arithmetic median of the grades given to the sample by the at least 3 subjects show a grading of 3 or better (i.e., between grade 1 and 3).

In embodiments, more than 3 subjects take part in the test, e.g. at least 6, at least 12, at least 24, at least 48 or at least 96.

## Shannon Index

The Shannon index, also known as Shannon's diversity index, Shannon-Wiener index, and (erroneously) Shannon-Weaver index is a measure originally proposed by Claude Shannon in 1948 to quantify the entropy (hence Shannon entropy) in strings of text. In present times, the Shannon-index is widely used in the area of ecology to describe the diversity of biomes.

The initial idea of the Shannon index was that the more letters there are, and the closer their proportional abundances in the string of interest, the more difficult it is to correctly predict which letter will be the next one in the string. The Shannon entropy quantifies the uncertainty (entropy or degree of surprise) associated with this prediction. It is most often calculated (including in this disclosure) as follows:

$$H' = - \sum_{i=1}^{R} p_i \ln p_i$$

where $p_i$ is the proportion of characters belonging to the i-th type of letter in the string of interest.

In ecology, $p_i$ is the proportion of individuals belonging to the i-th species in the dataset of interest. Thus, the Shannon entropy simply quantifies the uncertainty in predicting the species identity of an individual that is taken at random from the dataset. The higher this "uncertainty", the more diverse is the microbiome.

When all types in the dataset of interest are equally common, all $p_i$ values equal 1 / R, and the Shannon index hence takes the value ln(R). The more unequal the abundances of the types, the larger the weighted geometric mean of the p; values, and the smaller the corresponding Shannon entropy. If practically all abundance is concentrated to one type, and the other types are very rare (even if there are many of them), Shannon entropy approaches zero. When there is only one type in the dataset, Shannon entropy exactly equals zero (there is no uncertainty in predicting the type of the next randomly chosen entity).

PAST software v.3.01 can be used to calculate the Shannon index as published in H, Harper D, Ryan P. PAST-Palaeontological Statistics, ver. 1.89. Palaeontol Electron. 2001;4(1):1-9.

## Further definitions

The term "surface roughness" as used herein is a component of surface texture. It is quantified by the deviations in the direction of the normal vector of a real surface from its ideal form. If these deviations are large, the surface is rough; if they are small, the surface is smooth. In surface metrology, roughness is typically considered to be the high-frequency, short-wavelength component of a measured surface. When dealing with the roughness profile, Ra is referred to as defined in DIN EN ISO 4287:2010-07.

The term "water contact angle" quantifies the wettability of a solid surface by a liquid via the Young equation. The water contact angle can be measured according to DIN 55660-2:2011-12, chapter 5.2.2. using a static method with a drop volume of 2 $\mu$l.

The term "surface" as used in this disclosure denotes an area forming the outside part or uppermost layer of an article, i.e. usually the interface of the article and the surrounding atmosphere. The surface area of particulate surfaces (e.g. powders or granules) or of porous surfaces may be measured by B.E.T.-measurement according to DIN ISO 9277:2022.

**Embodiments**

In one embodiment, the microorganism, or spore thereof, and/or the metabolic biofilm has amylase activity, i.e., shows the conversion of starch into sugar by the action of enzymes such as amylase. Amylase activity can be measured using the well-known Phadebas test. Amylase activity can be represented by an absolute amylase activity score and/or a relative amylase activity ratio. Optionally, the microorganism, or spore thereof, and/or the metabolic biofilm has an absolute amylase activity score of at least 1, at least 2, at least 3, or at least 4. In an embodiment, the absolute amylase activity score may range up to 5, or up to 4. For example, the relative amylase activity ratio may range from 1 to 5, from 2 to 5, from 3 to 4, or it may be about 3.

Alternatively, or in addition, the microorganism, or spore thereof, and/or the metabolic biofilm may have an amylase activity larger than an amylase activity of DSM402. Optionally, the relative amylase activity ratio of at least 5:1, at least 6:1, at least 7:1, or at least 8:1. In an embodiment, the relative amylase activity ratio may range up to 100:1, up to 50:1, up to 30:1, or up to 20:1. For example, the relative amylase activity ratio may range from 5:1 to 100:1, from 6:1 to 50:1, from 7:1 to 30:1, or from 8:1 to 20:1.

In one embodiment, the microorganism, or spore thereof, and/or the metabolic biofilm has protease activity, i.e., has the activity to breakdown proteins into smaller polypeptides or amino acids. Protease activity can be measured using well-known protease tests. This property can be represented by an absolute protease activity score and/or a relative protease activity ratio. Optionally, the microorganism, or spore thereof, and/or the metabolic biofilm has an absolute protease activity score of at least 1, at least 2, at least 3, or at least 4. In an embodiment, the absolute protease activity score may range up to 5, or up to 4. For example, the relative amylase activity ratio may range from 1 to 5, from 2 to 5, from 3 to 4, or it may be about 3 or about 4.

Alternatively, or in addition, the microorganism, or spore thereof, and/or the metabolic biofilm may have a protease activity larger than a protease activity of DSM402. Optionally, the metabolic biofilm has a relative protease activity ratio of at least 1.2:1, at least 1.5:1, at least 2:1, at least 2.5:1, at least 3:1, at least 4:1, or at least 5:1. In an embodiment, the relative protease activity ratio may range up to 100:1, up to 40:1, up to 20:1, or up to 13:1. For example, the relative amylase activity ratio may range from 1.2:1 to 100:1, from 1.5:1 to 40:1, from 2:1 to 20:1, or from 4:1 to 13:1.

In an embodiment, the microorganism, or spore thereof, does not have a negative impact on the diversity of the microbiome of the biofilm and is not a biocide. This is reflected by the alpha-diversity according to the Shannon-index, wherein a Shannon-index above 1.5 or higher shows a good diversity of microorganisms. This means that a metabolic biofilm as formed by the microorganism or spore thereof may have a Shannon index of more than 1.5. When referring to the microorganism or spore thereof having a Shannon index of more than X, it is meant that the Shannon index of a biofilm as obtained after the wash and dry cycles according to the long-term polymer matrix test described hereinabove. Thus, in a further embodiment, the microorganism, or spore thereof, and/or the metabolic biofilm has an alpha-diversity reflected by a Shannon-index of at least 1.5, of at least 1.8, of at least 2.25, of at least 2.8, of at least 3, of at least 4, of at least 5, or of at least 6. The diversity and the type of the individual microorganism may be determined by CFU and gDNA determination.

In an embodiment, the microorganism, or spore thereof is applied to, or disposed on, a surface, wherein optionally

a. the surface is a surface of the built environment and/or a human-made article;

b. the surface is susceptible to having biofilms,

c. the surface has a surface roughness of at least 1 $\mu$m $R_a$;

d. the surface has a minimum hydrophilicity defined by a water contact angle of less than 80°;

e. the surface is selected from the group consisting of biologic, polymeric, organic or inorganic surface, and/or

f. application or disposal of the microorganism or sport is by injecting, impregnating, immersing, spraying, printing, calendaring, and/or dipping process followed by drying.

The surface may be surface of an article, particularly a human-made article, i.e. it may exclude articles found in nature, e.g. stones, trees, earth. In an embodiment, the article comprises the microorganism, or spore thereof and/or a metabolic biofilm. The microorganism, or spore thereof and/or the metabolic biofilm may disposed on a surface of said article. When reference is made to a "surface" herein, this may optionally refer to the surface of the article discussed herein.

The metabolic biofilm optionally may be present on a surface of the built environment and/or on a surface of the article. In general, all surfaces which are susceptible to having biofilms are included. In one embodiment, the microorganism, or spore thereof, is applied to or disposed on a surface, wherein preferably the surface may be characterized by a surface roughness of at least 1 $\mu$m Ra, at least 2 $\mu$m Ra, at least 3 $\mu$m Ra, at least 5 $\mu$m Ra, at least 8 $\mu$m Ra, at least 10 $\mu$m Ra, at least 12 $\mu$m Ra, or at least 15 $\mu$m Ra.

In one embodiment, the microorganism, or spore thereof, is applied to or disposed on a surface that has a minimum hydrophilicity defined by a water contact angle of less than 80°. In other embodiments a water contact angle of less than 60°, a water contact angle of less than 45°, a water contact angle of less than 30°, a water contact angle of less than 15° or less. In one embodiment the water contact angle is about 0° and the surface is considered hydrophilic.

The surface may be selected from the group consisting of a biologic, polymeric, organic and/or inorganic surface.

The article may be selected from

- a household article, optionally selected from shower curtains and cleaning articles for cosmetic use, such as make-up tissues, make-up cleaning pads (cosmetic pads), cotton swabs, etc.;

- a kitchen article, optionally selected from kitchen tools, kitchen-wares, dish clothes, brushes, scourer and the like;

- a fabric, optionally selected from textiles, fibers, clothes, garments, polymeric matrices (for example PU or cellulose), cellulosic materials, synthetic materials and/or man-made fibres, wool or animal hair material, or blends thereof; and/or

- a filter, such as an air filter.

In yet a further embodiment, the microorganism, or spore thereof, may be applied on or into a material of the article or surface comprising or consisting of

- synthetic fibers, including polyamide, polyester, polyvinyl alcohol, polyvinyl acetate, polyolefines, polycarbonates, glass, carbon, polylactic acid, polyacrylic, polyhydroxyalcanoates,;

- natural fibers, such as flax, linen, cotton, sisal, viscose, wool, kapok, hemp, chitosan, chitin; synthetic porous materials, such as polyurethane, polyvinyl acetate, polyvinyl alcohol, melamine, synthetic rubber; natural porous materials, such as cellulose, viscose, collagen, gelatin, glucomannan, natural rubber;

- chemical binders, such as polyacrylic, polyvinylacetate, phenol formaldehyde resins, urea formaldehyde resins, styrene butadiene rubbers;

- natural binders, such as lignin, tannins, proteins, pectins, natural rubber, starch, gelatin, cellulose derivatives;

- synthetic abrasives, such as acrylate, polyurethane, phenolic based resins;

- natural particles, such as grinded nutshells, silica, alumina, carbonate, pumice

- viscose, polyester, polyamide, polyvinyl acetate, polypropylene, cotton, cellulose or mixtures thereof; and/or

- combinations thereof.

In another embodiment, the concentration of the microorganism, or spore thereof, is determined on the surface and/or in a volume of the article.

The microorganism, or spore thereof, may be present on the surface in a concentration of about $1 \times 10^2$ to 1

$\times$ $10^{10}$ "colony forming units per surface" i.e., CFU/cm$^2$. In a further embodiment, the microorganism, or spore thereof, is present on the surface in a concentration of about $1 * 10^2$ to $1 * 10^9$ CFU/cm$^2$, in a concentration of about $1 * 10^4$ to $1 * 10^8$ CFU/cm$^2$, or in a concentration of about $1 * 10^6$ to $1 * 10^8$ CFU/cm$^2$, in yet further embodiment in a concentration of about $1 \times 10^7$ CFU/cm$^2$.

In case of volumes, the number of developing colonies is then represented as "colony forming units per volume", i.e., in CFU/cm$^3$.The microorganism, or spore thereof, may be present in the human-made article in a concentration of about $1 \times 10^2$ to $1 \times 10^{10}$ CFU/cm$^3$. In a further embodiment the microorganism, or spore thereof, is present in a concentration of about $1 * 10^3$ to $1 * 10^9$ CFU/cm$^3$, in a concentration of about $1 * 10^4$ to $1 * 10^8$ CFU/cm$^3$, or in a concentration of about $1 * 10^6$ to $1 * 10^8$ CFU/cm$^3$, in yet further embodiment in a concentration of about $1 \times 10^7$ CFU/cm$^3$.

In one embodiment, the concentration of the microorganism, or spore thereof, on the surface and/or in the volume can be determined by measuring the genomic DNA. Optionally, the gDNA is at least 1 ng gDNA / cm$^3$, at least 5 ng gDNA / cm$^3$, at least 10 ng gDNA / cm$^3$, at least 15 ng gDNA / cm$^3$, or at least 25 ng gDNA / cm$^3$.

In another embodiment the disclosure relates to a composition comprising the microorganism, or spore thereof, and in some embodiments at least one further excipient, such as for example a buffer, a salt, a preservative, a deposit aid, a pharmaceutical excipient and/or a carrier.

The composition may be in the form of a solid, semi-solid, gel, liquid, aerosol, aqueous solution, emulsion, granules, and/or powder. The compositions may comprise combinations or blends of two or more of the microorganisms, or spores thereof, described herein. Optionally, a combination or blend may comprise two or more species and/or two or more isolates. The isolates may include at least two, at least three or more isolates of spores or other vegetative cell state of the microorganism, or spore thereof.

The composition may also be a ready-to-use (in-use) composition. The composition may furthermore be an active cleaning base ingredient to be incorporated into other cleaning or washing compositions.

The composition may further comprise a "deposit aid", which facilitates the deposition to the surface of the built environment and/or a human-made article during a washing process, such as a laundry process, dishwasher process, or hand washing process. Such a deposit aid may be any excipient which improves the application, for example an emulsifier, a surfactant, a stabilizer, an adhesion aid, or the like.

The composition comprising the microorganism or spore thereof may be a washing agent, such as a dishwashing agent. Such a washing agent can be present as powdered or tablet solids, homogeneous solutions, or suspensions. It can in principle contain all known ingredients that are usual in such agents.

The composition can contain, in particular, builder substances, surface-active surfactants, peroxygen compounds, water-miscible organic solvents, enzymes, sequestering agents, electrolytes, pH regulators, and further adjuvants such as silver corrosion inhibitors, foam regulators, additional peroxygen activators, and colouring agents and/or fragrances.

In an embodiment, this disclosure includes a composition comprising the microorganism or spore thereof for use in automatic dishwashing methods, including so-called "3 in 1" products that combine the conventional cleaner, rinse agent, and regenerating-salt agents in one microbial agent.

Suitable in principle as builder components, in particular in the low-alkalinity cleaning agents, are all builders usually used in automatic dishwashing agents, for example polymeric alkali phosphates, which can be present in the form of their alkaline, neutral, or acid sodium or potassium salts. Examples thereof are tetrasodium diphosphate, disodium dihydrogen diphosphate, pentasodium triphosphate, so-called sodium hexametaphosphate, and the corresponding potassium salts, or mixtures of sodium and potassium salts.

Their quantities can be in the range of up to approximately 35 wt-%, based on the entire composition; however, in an embodiment, the compositions comprising the microorganism are optionally free of such phosphates. Further possible water-soluble builder components are, for example, organic polymers or natural or synthetic origin, chiefly polycarboxylates, which may act as co-builders especially in hard-water regions.

Optional builders are, for example, polyacrylic acids and copolymers of maleic acid anhydride and acrylic acid, as well as the sodium salts of said polymeric acids. Commercially available products are, for example, Sokalan® CP 5 and PA 30 of the BASF company. Among the polymers of natural origin usable as co-builders are, for example, oxidized starch and polyamino acids such as polyglutamic acid or polyaspartic acid.

Further possible builder components are naturally occurring hydroxycarboxylic acids such as, for example, mono-, dihydroxysuccinic acid, 3-hydroxypropionic acid, and gluconic acid. Among the builder components are the salts of citric acid, in particular sodium citrate. Optional sodium citrate forms are anhydrous trisodium citrate and trisodium citrate dihydrate. Trisodium citrate dihydrate can be used as a finely or coarsely crystalline powder.

Depending on the pH ultimately established in the composition comprising the microorganism or spore thereof according to this disclosure, the acids corresponding to the aforesaid co-builder salts can also be present.

Suitable oxygen-based bleaching agents are principally alkali perborate mono- or tetrahydrate and/or alkali percarbonate, sodium may be the preferred alkali metal. The use of sodium percarbonate has advantages especially in cleaning agents for tableware, since it has useful effects on corrosion properties on glassware. The oxygen-based bleaching agent may therefore be an alkali percarbonate, in particular sodium percarbonate. Additionally or, in particular, alternatively, known peroxycarboxylic acids, for example dodecanediperacid or phthalimidopercarboxylic acids, which if applicable can be substituted on the aromatic portion, can be contained.

The addition of small quantities of known bleaching-agent stabilizers such as, for example, phosphonates, borates or metaborates, and metasilicates, as well as magnesium salts such as magnesium sulfate, may furthermore be expedient. Because cleaning agents are usually utilized in an air atmosphere, the microbial agents according to this disclosure can also be free of bleaching agents, since the bleach catalysts already exhibit an effect in the presence of atmospheric oxygen.

Transition-metal salts or complexes known as bleach-activating active substances, and/or conventional bleach activators, i.e. compounds that, under perhydrolysis conditions, yield optionally substituted perbenzoic acid and/or peroxycarboxylic acids having 1 to 10 carbon atoms, in particular 2 to 4 carbon atoms, can be used; the presence of peroxygen-based bleaching agent may then be necessary, however.

The usual bleach activators cited above, which carry O-and/or N-acyl groups having the aforesaid number of carbon atoms, and/or which carry optionally substituted benzoyl groups, are suitable. Multiply acylated alkylenediamines, in particular tetraacetylethylendiamine (TAED), acylated glycolurils, in particular tetraacetyl glycoluril (TAGU), acylated triazine derivatives, in particular 1,5-diacetyl-2,4-dioxohexahydro-1,3,5-triazine (DADHT), acylated phenylsulfonates, in particular nonanoyl- or isononanoyloxybenzenesulfonate, acylated polyvalent alcohols, in particular triacetin, ethylene glycol diacetate, 2,5-diacetoxy-2,5-dihydrofuran, as well as acetylated sorbitol and mannitol, and acylated sugar derivatives, in particular pentaacetyl glucose (PAG), pentaacetyl fructose, tetraacetyl xylose, and octaacetyl lactose, as well as acetylated, optionally N-alkylated glucamine and gluconolactone, may be used. Combinations of conventional bleach activators can also be used.

Automatic dishwashing microbial agents optionally contain the usual alkali carriers such as, for example, alkali silicates, alkali carbonates, and/or alkali hydrogencarbonates. Included among the alkali carriers usually used are carbonates, hydrogencarbonates, and alkali silicates having a SiC / IV O molar ratio (M=alkali atom) from 1.5: 1 to 2.5: 1. Alkali silicates can be contained in quantities of up to 30 wt-% based on the entire microbial agent. It is optionally to dispense entirely with the use of the highly alkaline metasilicates as alkali carriers.

The alkali carrier system optionally used in the composition is a mixture of carbonate and hydrogencarbonate, optionally sodium carbonate and hydrogencarbonate, which is contained in a quantity of up to 60 wt-%, optionally 10 wt-% to 40 wt-%. The ratio of carbonate to hydrogencarbonate varies depending on the pH that is ultimately desired, although an excess of sodium hydrogencarbonate is usually used, so that the weight ratio between hydrogencarbonate and carbonate is generally 1:1 to 15:1.

In a further embodiment of the composition, 20 wt-% to 40 wt-% water-soluble organic builders, in particular alkali citrate, 5 wt-% to 15 wt-% alkali carbonate, and 20 wt-% to 40 wt-% alkali disilicate are contained.

The composition can also, if applicable, contain surfactants, in particular low-foaming non-ionic surfactants that may provide better detachment of grease containing stains and serve as a wetting agent and, if applicable, as a granulating adjuvant in the context of manufacture of the composition comprising the microorganism. Their quantity can be up to 10 wt-%, in particular up to 5 wt-%, and is optionally in the range from 0.5 wt-% to 3 wt-%.

These compounds include, optionally, $C_{12}$-$C_{18}$ alkylpolyethylene-glycolpolypropylene-glycol ethers having respectively up to 8 units of ethylene oxide and propylene oxide units in the molecule. It is also possible, however, to use other known low-foaming non-ionic surfactants such as, for example, $C_{12}$-$C_{18}$ alkylpolyethylene glycol-polybutylene glycol ethers having respectively up to 8 units of ethylene oxide and butylene oxide units in the molecule, endcapped alkylpolyalkylene glycol mixed ethers, and the foaming but environmentally attractive $C_8$-$C_{14}$ alkyl polyglucosides having a degree of polymerization from approximately 1 to 4 (e.g. APG® 225 and APG® 600), and/or $C_{12}$-$C_{14}$ alkyl polyethylene glycols having 3 to 8 ethylene oxide units in the molecule.

Likewise suitable are surfactants from the family of the glucamides such as, for example, alkyl-N-methylglucamides, in which the alkyl part optionally derives from a fatty alcohol having a carbon chain length of $C_6$ to $C_{14}$.

It is in some cases advantageous if the above-described surfactants are used as mixtures, for example the combination of alkyl polyglycoside with fatty alcohol ethoxylates, or glucamide with alkyl polyglycosides.

Optional silver corrosion protection agents are organic disulfides, divalent phenols, trivalent phenols, optionally substituted benzotriazole, salts and/or complexes of manganese, titanium, zirconium, hafnium, vanadium, cobalt, or cerium, in which the aforesaid metals are present in one of the oxidations states II, III, IV, V, or VI.

In addition, the composition according to the present disclosure can contain enzymes such as proteases, amylases, pullulases, cutinases, and lipases, for example proteases such as BLAP®, Optimase®, Opticlean®, Maxacal®, Maxapem®, Esperase®, and/or Savinase®, amylases such as Termamyl®, Amylase-LT®, Maxamyl®, and/or Duramyl®, lipases such as Lipolase®, Lipomax®, Lumafast®, and/or Lipozym®.

The enzymes that are used if applicable can be adsorbed onto carrier substances and/or embedded into encasing substances in order to protect them from premature inactivation. They are contained in the composition in quantities optionally not above 2 wt-%, in particular from 0.1 wt-% to 0.7 wt-%.

If the compositions foam excessively upon use, they can also include up to 6 wt-%, optionally approximately 0.5 wt-% to 4 wt-%, of a foam-suppressing compound, optionally from the group of the silicone oils, mixtures of silicone oil and hydrophobized silicic acid, paraffins, paraffin-alcohol combinations, hydrophobized silicic acid, the bis-fatty acid amides, and other known defoamers obtainable commercially.

Further optional ingredients in the composition are, for example, perfume oils.

Among the organic solvents usable in the composition, especially when present in liquid or paste form, are alcohols having 1 to 4 carbon atoms, in particular methanol, ethanol, isopropanol, and tert.-butanol, diols having 2 to 4 carbon atoms, in particular ethylene glycol and propylene glycol, as well as mixtures thereof, and the ethers derivable from the aforesaid compound classes.

Water-miscible solvents of this kind are present in the compositions comprising the microorganism according to the present disclosure at optionally no more than 20 wt-%, in particular from 1 wt-% to 15 wt-%.

In order to establish a desired pH that does not of itself result from the mixture of the other components, the composition can contain system-compatible and environmentally compatible acids, for example citric acid, acetic acid, tartaric acid, malic acid, lactic acid, glycolic acid , succinic acid, glutaric acid, and/or adipic acid, but also mineral acids, in particular sulfuric acid, or alkali hydrogensulfates or bases, in particular ammonium hydroxides or alkali hydroxides pH regulators of this kind are contained in the composition optionally at no more than 10 wt-%, in particular from 0.5 wt-% to 6 wt-%.

Manufacture of the solid composition presents no difficulties and can in principle be effected in known fashion, for example by spray drying or granulation, the peroxygen compound and bleach catalyst optionally being separately added later.

If applicable, the bleach catalyst is mixed with further raw materials and/or compounds, and the mixture is then compressed into tablets or phases thereof.

The composition in the form of aqueous solutions or those containing other usual solvents may be manufactured by mixing the ingredients, which can be placed, in substance or as a solution, into an automatic mixer.

The composition may be present as powdered, granular, or tablet preparations that can be manufactured in known fashion, for example by mixing, granulating, roller compacting, and/or by spray drying of the thermally insensitive components and mixing in the more-sensitive components, included among which are, in particular, enzymes, bleaching agents, and the bleach catalyst.

For the manufacture of the composition in tablet form, it is optional to proceed in such a way that all the constituents, or all the constituents provided for use together in one phase of the tablet, are mixed together in a mixer, and the mixture, or the mixtures in succession, are compressed by means of conventional tablet presses, for example eccentric presses or rotary tablet presses, at compression pressures in the range from $200 \times 10^5$ Pa to $1500 \times 10^5$ Pa.

Break-resistant tablets that are nevertheless sufficiently rapidly soluble under the utilization conditions, having flexural strength values normally above 150 N, are thereby obtained without difficulty.

A tablet manufactured in this fashion optionally has a weight from 15 g to 40 g, in particular from 20 g to 30 g. It can be round, for example with a diameter from 35 mm to 40 mm, or can have any other desired shape, for example rectangular with optionally rounded edges.

Compositions in the form of non-dusting, shelf-stable, pourable powders and/or granulates having high bulk densities in the range from 800 to 1000 g/L can be manufactured by mixing the builder components with at least a portion of liquid mixture components in a first partial step of the method, with an increase in the bulk density of this premixture, and then, if desired after intervening drying, combining the further constituents of the agent, among them the microbial agent, with the premixture thus obtained.

Compositions for cleaning tableware can be used in both household and commercial dishwashers. They are added manually or by means of suitable metering devices.

Description of the Figures

**Figure 1** - Standard curve depicting the relationship of α-amylase activity (U/l) and absorption in an amylase activity test.

**Figure 2** - Standard curve depicting the relationship of Subtilisin A activity and absorption in a protease test, the standard curve is linear up to an absorbance of 1.00.

Examples

**Example 1: Determination of biofilm establishment**

Multiple candidate microorganisms and spores thereof were investigated for their capability of forming a metabolic biofilm. Except #18 (Priestia megaterium), all candidates were B. subtilis.

PU foam cubes (1x1x1 cm, ca. 25 mg) were distributed into single wells of 24-deepwell plates (DWP) and sterilized by autoclaving for 20 min at 121°C. Each well was subsequently filled with 2 ml medium (skim milk powder 0.8 g/L, soluble starch 4 g/L, olive oil 0.6 g/L, sterilized by autoclaving). After 5 min. incubation, each cube was then picked up with tweezers and transferred into an empty well of a new DWP. Each cube was subsequently inoculated (4 replicates per candidate strain) by pipetting 10 $\mu$l spores suspension (1E+05 CFU, in PBS buffer) onto the surface, non-inoculated cubes served as control. Inoculated cubes are incubated at 20°C for 7 days. After 2 and 4 days within the 7 day period, medium was again applied to each well as above and cubes were subsequently transferred to a new DWP.

After 7 days, cubes were washed by applying 4 ml saline (0.9%) into each well and subsequent transfer with tweezers into a new DWP. This step was repeated two times. After washing, 2 ml of PrestoBlue (Thermo Fisher Scientific) dye solution (10 % in medium) was applied to the cubes in each well and the DWP was incubated for 6 hours at 37°C. Metabolic activity of biofilms was assessed by transferring 150 $\mu$l of each well into a microtitre plate (e.g., Costar 3904, Corning) and fluorescence detection (Fluostar, BMG) with the following parameters: Excitation wavelength 440-412 nm, emission wavelength 590 nm, gain 15, prior mixing (5 sec, 1 mm orbital). The mean values (MV) obtained (arbitrary fluorescence units) of suitable candidates should reach a minimum of 30.000 (after subtraction of MV of non-inoculated control) or a minimum of 2x mean values obtained for control strain (DSM402) treated in the same way. Microorganisms scoring at least 2x mean values obtained for control strain (DSM402) are considered capable of forming a metabolic biofilm.

Table 3: Fluorescence intensities (arbitrary units, AU) of biofilms inoculated from spores and grown in PU cubes for 7 days

| Strain | #18 (AU) - (ratio) | | #50 (AU) - (ratio) | | #46 (AU) - (ratio) | | DSM402 (AU) - (ratio) | |
|---|---|---|---|---|---|---|---|---|
| Replicate 1 | 39868 | 16.7 | 13799 | 5.8 | 30947 | 13 | 2397 | 1 |
| Replicate 2 | 52591 | 5.1 | 35926 | 3.5 | 52772 | 5.2 | 10240 | 1 |
| Replicate 3 | 39302 | 4.3 | 41076 | 45 | 35054 | 3.9 | 9082 | 1 |
| Replicate 4 | 38357 | 6.3 | 40917 | 6.7 | 3289 | 0.5 | 6104 | 1 |
| MV | 42530 | 6.1 | 32930 | 4.7 | 30516 | 4.4 | 6956 | 1 |

| Strains #18, #50 and #46 develop biofilms with an average metabolic activity of a fluorescence of at least 2-times higher intensity as compared to DSM-402. |
|---|

**Example 2: Amylase activity**

PU foam cubes (1x1x1cm, ca. 25 mg) were distributed into single wells of 24 well deepwell plates (DWP) and sterilized by autoclaving for 20 min at 121°C. Each well was subsequently filled with 2 ml medium (Bacto Tryptone 10 g/L, NaCl 5 g/L, Bacto Yeast Extract 5 g/L, sterilized by autoclaving; glycerol 1%, $MnSO_4$ 0.1 mM, filter sterilized and supplemented after autoclaving). After 5 min incubation, each cube was then picked up with tweezers and transferred into an empty well of a new DWP. Each cube was subsequently inoculated (4 replicates per candidate strain) by pipetting 10 $\mu$l spores suspension (1E+05 CFU, in PBS buffer) onto the surface, non-inoculated cubes served as sterile control. Inoculated cubes were incubated at 20°C. After 72 hours, cubes were washed by applying 4 ml saline (0.9%) into each well and subsequent transfer with tweezers into a new DWP. This step was repeated two times. After washing, 2.5 ml of Phadebas solution (9 Phadebas tablets were dissolved in 65 ml of a 2.4 g/L starch solution, sterilized by autoclaving) are added to each well. The DWP is incubated at 37°C on a Titramax at shaking speed 7 for 2 hours. From each well 200 $\mu$l samples are subsequently transferred into 1.5 ml reaction tubes and centrifuged (5 min, 1500 g). Supernatant samples (100 $\mu$l) were transferred into a 96 well microtitre plate (MTP) and the absorbance is measured at 620 nm against deionized water (when absorbance exceeds the measuring capability of the photometer, supernatant is diluted 10-fold using deionized water).

Table 4: Absorbance values (620nm) of amylase assays using biofilms inoculated from spores and grown in PU cubes for 72 hours.

| Strain | #50 (Abs.) | #50 (ratio) | DSM402 (Abs.) | DSM402 (ratio) |
|---|---|---|---|---|
| Replicate 1 | 0.501 | 6.5 | 0.077 | 1 |
| Replicate 2 | 0.394 | 13.1 | 0.030 | 1 |
| Replicate 3 | 0.529 | 6.8 | 0.078 | 1 |
| Replicate 4 | 0.443 | 13 | 0.034 | 1 |
| **MV** | **0.467** | **8.5** | **0.055** | **1** |

## Example 3: Protease activity

PU foam cubes (1x1x1cm, ca. 25 mg) were distributed into single wells of 24 well deepwell plates (DWP) and sterilized by autoclaving for 20 min at 121°C. Each well was subsequently filled with 2 ml medium (skim milk powder 0.8 g/L, soluble starch 4 g/L, olive oil 0.6 g/L, sterilized by autoclaving). After 5 min incubation, each cube was then picked up with tweezers and transferred into an empty well of a new DWP. Each cube was subsequently inoculated (4 replicates per candidate strain) by pipetting 10 $\mu$l spores suspension (1E05 CFU, in PBS buffer) onto the surface, non-inoculated cubes served as control. Inoculated cubes were incubated at 20°C for 7 days. After 2 and 4 days, medium was again applied to each well as above and cubes were subsequently transferred to a new DWP. After 7 days, cubes were washed by applying 4 ml saline (0.9%) into each well and subsequent transfer with tweezers into a new DWP. This step was repeated two times. After washing, 2.5 ml of Azo-Casein (Megazyme, Ireland) solution were added to each well. Azo-Casein solution was prepared by mixing 26 ml medium (as above) and 39 ml Azo-Casein (0.8 g Azo-Casein powder reconstituted according to Manufacturer's instructions). The DWP was incubated at 37°C on a Titramax at shaking speed 7 for 6 hours. From each well 150 $\mu$l samples were subsequently transferred into 1.5 ml reaction tubes, 450 $\mu$l TCA (5%) were added, mixed by inverting and the tubes were centrifuged (5 min, 1500 g). Supernatant samples (150 $\mu$l) were transferred into a 96 well microtitre plate (MTP) and the absorbance was measured at 440 nm against deionized water (when absorbance exceeds the measuring capability of the photometer, supernatant is diluted 10-fold using deionized water).

Table 5: Absorbance values (440 nm) of protease assays using biofilms inoculated from spores and grown in PU cubes for 7 days.

| Strain | #18 (Abs.) | #18 (ratio) | DSM402 (Abs.) | #DSM402 (ratio) |
|---|---|---|---|---|
| Replicate 1 | 0.509 | 4.3 | 0.119 | 1 |
| Replicate 2 | 0.520 | 4.4 | 0.118 | 1 |
| Replicate 3 | 0.409 | 4.17 | 0.098 | 1 |
| Replicate 4 | 0.629 | 10 | 0.063 | 1 |
| **MV** | **0.517** | **5.17** | **0.100** | **1** |

## Example 4: Long-term polymer-matrix test: Amylase activity

A long-term polymer matrix test was performed in order to evaluate the presence of amylase activity after simulated use of the article, e.g. in the context of a household article with repeated wet and dry cycles. Initially, the long-term polymer matrix test was performed as described hereinabove, i.e. steps a. through d. After step d., sponges were harvested from the long-term polymer-matrix test by letting them dry completely without washing for 2 days at 22-25°C. Sponges can subsequently be stored in an airtight container for up to 4 weeks.

For enzyme assessment, dried sponges were divided into 8 equal parts and each 1/8 fragment was reactivated by immersing in 50 ml medium (skim milk powder 0.8 g/L, soluble starch 4 g/L, olive oil 0.6 g/L, sterilized by autoclaving). After incubation for 5 min, sponges were squeezed and air dried for 2 days at 22-25°C. Each 1/8 fragment was cut into 4 equal parts which were transferred into a 100 ml glass shake flask. Each shake flask was filled with 15 ml deionized water, 10 ml soluble starch solution (4 g/L, sterilized by autoclaving) and 3 Phadebas tablets (Phadebas AB, Sweden) were added. Shake flasks were incubated at 37°C, 250 rpm for 16 hours. From each shake flask, 500 $\mu$l liquid was transferred into a 1.5 ml tube and centrifuged at 16000xg for 2 min. 100 $\mu$l of supernatant was transferred into a 96-well microtitre plate (MTP) and the absorbance was measured at 620 nm against deionized water (when absorbance exceeds the measuring capability of the photometer, supernatant is diluted 10-fold using deionized water). The relative activities of sponges inoculated with candidates are calculated as follows: absorbance (inoculated sponge) / absorbance (non-inoculated sponge).

Table 6: Relative amylase activities

| Control (non-inoculated sample) = 1, candidates #14, #18, #38, #42, #46, #50 inoculated before the start of test, harvested after 6 weeks for long-term polymer matrix test. | | | | | | | |
|---|---|---|---|---|---|---|---|
| **Strain Inoculum** | **Control** | **#14** | **#18** | **#38** | **#42** | **#46** | **#50** |
| MV | 100 | 1.53 | 3.86 | 2.93 | 1.62 | 1.23 | 20.68 |

**Example 5**: **Long-term polymer-matrix test: Protease activity**

A long-term polymer matrix test was performed in order to evaluate the presence of protease activity after simulated use of the article, e.g. in the context of a household article with repeated wet and dry cycles. Initially, the long-term polymer matrix test was performed as described hereinabove, i.e. steps a. through d. After step d., sponges were harvested from the long-term polymer-matrix test by letting them dry completely without washing for 2 days at 22-25°C. Sponges can subsequently be stored in an airtight container for up to 4 weeks.

For enzyme assessment, dried sponges were divided into 8 equal parts and each 1/8 fragment was reactivated by immersing in 50 ml medium (skim milk powder 0.8 g/L, soluble starch 4 g/L, olive oil 0.6 g/L, sterilized by autoclaving). After incubation for 5 min, sponges were squeezed and air dried for 2 days at 22-25°C. Each 1/8 fragment was cut into 4 equal parts which were transferred into a 100 ml glass shake flask. Each shake flask was filled with 25 ml medium (skim milk powder 0.8 g/L, soluble starch 4 g/L, olive oil 0.6 g/L, sterilized by autoclaving) and incubated at 28°C 250 rpm for 16 hours. 15 ml of the liquid was removed from each shake flask and 15 ml azo-casein solution (2% prepared according to Manufacturer's instructions, Megazyme, Ireland) were added. The shake flasks were incubated at 37°C 250 rpm for 2 hours. 150 $\mu$l liquid from each shake flask was subsequently filled into a 1.5 ml tube, 450 $\mu$l TCA (5%) were added and samples were incubated for 5 min at room temperature. After centrifugation at 16000xg for 2 min, 200 $\mu$l supernatant were transferred into a 96-well microtitre plate (MTP) and the absorbance measured at 440 nm against deionized water (when absorbance exceeds the measuring capability of the photometer, supernatant is diluted 10-fold using deionized water). The relative activities of sponges inoculated with candidates are calculated as follows: absorbance (inoculated sponge) / absorbance (non-inoculated sponge).

Table 7: Relative protease activities

| Control (non-inoculated sample) = 1; candidates #14, #18, #38, #42, #46, #50 inoculated before the start of test, harvested after 6 weeks for long-term polymer matrix test. | | | | | | | |
|---|---|---|---|---|---|---|---|
| **Strain Inoculum** | **Control** | **#14** | **#18** | **#38** | **#42** | **#46** | **#50** |
| MW | 100 | 0.67 | 2.56 | 1.09 | 1.45 | 1.71 | 1.18 |

**Claims**

1. A microorganism, or spore thereof, capable of forming a metabolic biofilm.

2. The microorganism, or spore thereof according to claim 1, wherein the microorganism, or spore thereof is applied to, or disposed on, a surface, wherein optionally

   a. the surface is a surface of the built environment and/or a human-made article;
   b. the surface is susceptible to having biofilms,
   c. the surface has a surface roughness of at least 1 $\mu$m $R_a$;
   d. the surface has a minimum hydrophilicity defined by a water contact angle of less than 80°;
   e. the surface is selected from the group consisting of biologic, polymeric, organic or inorganic surface, and/or
   f. application or disposal of the microorganism or sport is by injecting, impregnating, immersing, spraying, printing, calendaring, and/or dipping process followed by drying.

3. The microorganism, or spore thereof, according to any of the previous claims, wherein the metabolic biofilm has a metabolic activity of at least 2-fold as compared to a standard organism *Bacillus subtilis* DSM-No. 402.

4. The microorganism, or spore thereof, according to any of the previous claims, wherein the metabolic biofilm has amylase activity, wherein optionally

    a. the metabolic biofilm has an absolute amylase activity score of at least 1 and/or
    b. the metabolic biofilm has a relative amylase activity ratio of at least 5:1.

5. The microorganism, or spore thereof, according to any of the previous claims, wherein the metabolic biofilm has protease activity, wherein optionally

    a. the metabolic biofilm has an absolute protease activity score of at least 1, and/or
    b. the metabolic biofilm has a relative protease activity ratio of at least 1.2:1.

6. The microorganism, or spore thereof, according to any of the previous claims, wherein the metabolic biofilm has an alpha-diversity reflected by a Shannon-index of at least 1.5, at least 1.8, or at least 2.

7. The microorganism, or spore thereof, according to any of the previous claims, wherein the microorganism, or spore thereof, is selected of a group consisting of

    at least one member of the *Bacillus subtilis* group, wherein the member *Bacillus subtilis group* is selected from a member of the group consisting of *Bacillus amyloliquefaciens, Bacillus tequilensis, Bacillus subtilis, Bacillus atrophaeus, Bacillus vallismortis, Bacillus licheniformis, Bacillus sonorensis* and *Bacillus mojavensis;* and/or a member of the *Priestia* (former *Bacillus)* megaterium, and/or a member of *Bacillus velezensis,* and/or a member of *Bacillus pumilus,* and/or a mixture or combination thereof.

8. The microorganism, or spore thereof, according to claim 7, wherein the at least one microorganism, or spore thereof, is either included in the list of QPS status recommended biological agents for safety risk assessments as published by the European food safety authority as QPS-EFSA List 10.2903/j.efsa.2022.7408, or fulfils all the requirements to be included in said list.

9. The microorganism, or spore thereof, according to any of the previous claims, wherein the microbial organism, or spore thereof, has passed at least one test selected from

    a. a hot washing test with a ratio of less than 100,
    b. a heat test with a ratio of less than 100,
    c. a chemical stress test with a ratio of less than 100,
    d. an amylase polymer matrix test with a ratio of at least 1.5, and/or
    e. a protease polymer matrix test with a ratio of at least 1.5.

10. The microorganism, or spore thereof, according to any of claims 2 to 9, wherein the microorganism, or spore thereof, is present on at least one surface-section of 1 $cm^2$ in a concentration of about $1 * 10^2$ to $1 * 10^{10}$ $CFU/cm^2$ and/or in at least one volume-section of 1 $cm^3$ of the human-made article in a concentration of about $1 * 10^2$ to $1 * 10^{10}$ $CFU/cm^3$.

11. The microorganism, or spore thereof, according to one or more of claims 2 to 10, wherein the material of the article or surface comprises or consists of

    - synthetic fibers, including polyamide, polyester, polyvinyl alcohol, polyvinyl acetate, polyolefines, polycarbonates, glass, carbon, polylactic acid, polyacrylic, polyhydroxyalcanoates;
    - natural fibers, such as flax, linen, cotton, sisal, viscose, wool, kapok, hemp, chitosan, chitin; synthetic porous materials, such as polyurethane, polyvinyl acetate, polyvinyl alcohol, melamine, synthetic rubber; natural porous materials, such as cellulose, viscose, collagen, gelatin, glucomannan, natural rubber;
    - chemical binders, such as polyacrylic, polyvinylacetate, phenol formaldehyde resins, urea formaldehyde resins, styrene butadiene rubbers;

- natural binders, such as lignin, tannins, proteins, pectins, natural rubber, starch, gelatin, cellulose derivatives;
- synthetic abrasives, such as acrylate, polyurethane, phenolic based resins;
- natural particles, such as grinded nutshells, silica, alumina, carbonate, pumice
- viscose, polyester, polyamide, polyvinyl acetate, polypropylene, cotton, cellulose or mixtures thereof; and/or
- combinations thereof.

12. Method of producing a microorganism, or spore thereof, capable of forming a metabolic biofilm, comprising the steps of:

a. Selecting a biofilm forming microorganism, or spore thereof,
b. Producing an expression vector comprising at least one gene of which the gene-product has a metabolic activity selected from amylase and/or protease activity,
c. Transforming the microorganism, or spore thereof, with the expression vector either stably and/or transiently,
d. Isolating the microorganism, or spore thereof, thereby producing a microorganism, or spore thereof, capable of forming a metabolic biofilm.

13. The method according to claim 12, wherein the at least one microorganism, or spore thereof, is applied in a further step e. to a surface by injecting, impregnating, immersing, spraying, printing, calendaring, and/or dipping process, optionally followed by drying.

14. Use of the microorganism, or spore thereof, according to any of claims 1 to 11 for

inhibiting and/or reducing a health risk and/or discomfort in the environment by producing a metabolic biofilm in households, during food processes, during fermentation processes, during pharmaceutical processes, in public health environments, and/or
facilitating malodour inhibition by producing a metabolic biofilm.

15. Use of a microorganism, or spore thereof, capable of forming a metabolic biofilm on a surface, wherein malodour is inhibited and/or reduced as compared to a surface not comprising the metabolic biofilm, corresponding to an odour difference of at least 0.3 grades, at least 0.5 grades, or at least 1.0 grade in the VDA odour test.

Figure 1

Figure 2

Europäisches
Patentamt

European
Patent Office

Office européen
des brevets

# EUROPEAN SEARCH REPORT

Application Number

EP 22 21 6428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/100854 A1 (GOODMAN STEVEN D [US] ET AL) 8 April 2021 (2021-04-08) | 1-3,6, 8-11,14 | INV. A01N63/20 A01N63/22 A01P1/00 C11D3/38 |
| Y | * paragraphs [0006] - [0009], [0010] - [0013], [0051] - [0054], [0083] - [0086], [0125] - [0126], [0178], [0315], [0320] * | 4,5,7, 12,13 | |
| E | EP 4 108 751 A1 (FREUDENBERG CARL KG [DE]) 28 December 2022 (2022-12-28)  * paragraphs [0001] - [0004], [0010], [0013], [0026] - [0028], [0037], [0039], [0041] - [0043], [0061] - [0066], [0076] - [0085] * * paragraphs [0098] - [0102] * | 1-3, 6-11,14, 15 | |
| Y | CN 111 826 323 A (FUJIAN LUODONG BIO TECH CO LTD) 27 October 2020 (2020-10-27) * paragraphs [0006] - [0007], [0010] - [0013], [0025], [0028] - [0029], [0062] - [0066] * | 4,5,7, 12,13 | |
| X | WO 2010/028460 A2 (KERSTENS PETER [BE]) 18 March 2010 (2010-03-18) * page 1, line 5 - line 16 * * page 1, line 38 - page 2, line 16 * * page 2, line 23 - line 41 * * page 3, line 15 - line 38 * * page 4, line 22 - line 26 * | 1-3,6-8, 11,14 | TECHNICAL FIELDS SEARCHED (IPC)  A01N A01P C12R C11D |
| A | WO 2021/195776 A1 (LULULEMON ATHLETICA CANADA INC [CA]) 7 October 2021 (2021-10-07) * claims 1, 10 * | 1-15 | |

-/--

The present search report has been drawn up for all claims

| Place of search | Date of completion of the search | Examiner |
|---|---|---|
| Munich | 21 June 2023 | Zanobini, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 21 6428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2012/105805 A2 (CJ CHEILJEDANG CORP [KR]; YANG SI YONG [KR] ET AL.) 9 August 2012 (2012-08-09) * paragraphs [0039] – [0046], [0057] – [0060], [0105] – [0108] * ----- | 1-15 | |

TECHNICAL FIELDS
SEARCHED (IPC)

The present search report has been drawn up for all claims

| Place of search | Date of completion of the search | Examiner |
|---|---|---|
| Munich | 21 June 2023 | Zanobini, Alessandra |

EPO FORM 1503 03.82 (P04C01)

## ANNEX TO THE EUROPEAN SEARCH REPORT
## ON EUROPEAN PATENT APPLICATION NO.

EP 22 21 6428

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2023

| Patent document cited in search report | | Publication date | Patent family member(s) | | Publication date |
|---|---|---|---|---|---|
| US 2021100854 | A1 | 08-04-2021 | US 2018000878 A1 | | 04-01-2018 |
| | | | US 2021100854 A1 | | 08-04-2021 |
| EP 4108751 | A1 | 28-12-2022 | EP 4108751 A1 | | 28-12-2022 |
| | | | WO 2022268985 A1 | | 29-12-2022 |
| CN 111826323 | A | 27-10-2020 | NONE | | |
| WO 2010028460 | A2 | 18-03-2010 | AU 2009291526 A1 | | 18-03-2010 |
| | | | BE 1018125 A5 | | 04-05-2010 |
| | | | CN 102137594 A | | 27-07-2011 |
| | | | US 2010040784 A1 | | 18-02-2010 |
| | | | WO 2010028460 A2 | | 18-03-2010 |
| | | | ZA 201101929 B | | 27-06-2012 |
| WO 2021195776 | A1 | 07-10-2021 | CA 3171419 A1 | | 07-10-2021 |
| | | | CN 115380110 A | | 22-11-2022 |
| | | | EP 4127159 A1 | | 08-02-2023 |
| | | | US 2023115063 A1 | | 13-04-2023 |
| | | | WO 2021195776 A1 | | 07-10-2021 |
| WO 2012105805 | A2 | 09-08-2012 | CN 103403146 A | | 20-11-2013 |
| | | | KR 20120088437 A | | 08-08-2012 |
| | | | WO 2012105805 A2 | | 09-08-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Probiotics Antimicrob. Proteins,* September 2019, vol. 11 (3), 731-747

- **HARPER D ; RYAN P.** PAST-Palaeontological Statistics, ver. 1.89. *Palaeontol Electron.,* 2001, vol. 4 (1), 1-9